(19) Europäisches Patentamt
European Patent Office
Office européen des brevets

(11) Veröffentlichungsnummer: **0 154 805**
**B1**

(12) # EUROPÄISCHE PATENTSCHRIFT

(45) Veröffentlichungstag der Patentschrift: **01.07.87**

(51) Int. Cl.⁴: **C 08 J 9/06**, C 08 L 69/00, C 07 D 271/10

(21) Anmeldenummer: **85101171.8**

(22) Anmeldetag: **05.02.85**

(54) **Verfahren zum Verschäumen von thermoplastischen aromatischen Polycarbonaten.**

(30) Priorität: **15.02.84 DE 3405418**

(43) Veröffentlichungstag der Anmeldung: **18.09.85 Patentblatt 85/38**

(45) Bekanntmachung des Hinweises auf die Patenterteilung: **01.07.87 Patentblatt 87/27**

(84) Benannte Vertragsstaaten: **DE FR GB IT NL**

(56) Entgegenhaltungen: **EP-A-0 130 447**

(73) Patentinhaber: **BAYER AG, Konzernverwaltung RP Patentabteilung, D-5090 Leverkusen 1 Bayerwerk (DE)**

(72) Erfinder: **Hammer, Heinz, Dr., Rybnikerstrasse 12, D-5000 Köln 80 (DE)**
Erfinder: **Kircher, Klaus, Dr., Alfred- Kubin-Strasse 3, D-5090 Leverkusen (DE)**
Erfinder: **Meyer, Rolf- Volker, Dr., Buchheimerstrasse 23, D-4150 Krefeld (DE)**

EP 0 154 805 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Das Verschäumen von thermoplastischen aromatischen Polycarbonaten mit Treibmitteln ist bekannt (siehe beispielsweise Kunststoffe 62 (10), 687 (1972)).

Es ist auch bekannt, als Treibmittel eine Treibmittelkombination einzusetzen (siehe beispielsweise DE-A 2 441 418).

Es ist außerdem bekannt, die Verarbeitungstemperatur von Polycarbonat für die Herstellung eines Treibmittelkonzentrates herabzusetzen (siehe beispielsweise US-Patent 4 313 873 und EP-A 0 007 437).

Aufgabe der vorliegenden Erfindung ist es nun, ein Treibmittelsystem bei der Verschäumung von Polycarbonaten einzusetzten, das den Abbau der Kohlensäureesterbindungen im Polycarbonat nicht zu sehr verursacht, das zweitens eine relativ hohe Einarbeitungstemperatur erlaubt, um lagerfähige Treibmittel-haltige Polycarbonat-Granulate herzustellen, und das drittens bei der Verarbeitung des Polycarbonats zu Schaumkörpern eine möglichst geringe Verarbeitungstemperatur ermöglicht. Denn für die Verschäumung von Polycarbonat ist häufig nur eine Verarbeitungstemperatur von maximal 290° C-300° C möglich. Ein bei dieser Verarbeitungstemperatur ausreichend funktionsfähiges Treibmittel muß bereits bei einer Massetemperatur von 265° C-275° C eine ausreichende Zersetzungsgeschwindigkeit haben.

Berücksichtigt man einen Sicherheitsabstand von mindestens 25° C zwischen dieser Zersetzungstemperatur und der Herstellungstemperatur eines Treibmittelkonzentrates, so sind derartige Treibmittel bei maximal 240° C-250° C zur Herstellung von Treibmittelkonzentraten im Polycarbonat geeignet.

Um nun aber ein Treibmittel verwenden zu können, dessen Zersetzung erst oberhalb von 275° C in ausreichendem Maß stattfindet, bedarf es eines Zusatzstoffes, der die Zersetzung des Treibmittels und damit die Verarbeitungstemperatur der Treibmittelkonzentrate zu Polycarbonatschäumen herabsetzt.

Überraschend wurde nun ein Verfahren zum Verschäumen von aromatischen thermoplastischen Polycarbonaten gefunden, das bei Verarbeitungstemperaturen von 270° C-320° C anwendbar ist, das den gewünschten Anforderungen entspricht, und das dadurch gekennzeichnet ist, daß man als Treibmittel Verbindungen der Formel I

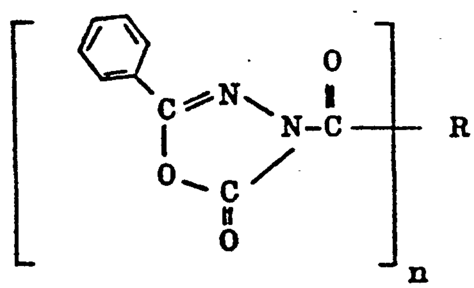

(I)

worin n = 1 und R $C_1$-$C_{18}$-Alkoxy, $C_6$-$C_{12}$-Cycloalkoxy, $C_6$-$C_{18}$-Aryl, $C_6$-$C_{12}$-Aryloxy, $C_7$-$C_{18}$-Aralkyl, $C_7$-$C_{18}$-Aralkoxy, $C_7$-$C_{18}$-Alkaryl und $C_7$-$C_{18}$-Alkaryloxy bedeuten oder

worin n = 2 und R $C_6$-$C_{18}$-Arylen, $C_6$-$C_{18}$-Arylen-dioxy, Isopropyliden-bis-(phenylen-oxy) und $C_1$-$C_5$-alkylen bedeuten oder eine Einfachbindung ist,

und die außerdem eine Zersetzungstemperatur in Substanz von mindestens 265° C, vorzugsweise von mindestens 275° C haben müssen, in Mengen von 0,02 bis 5 Gew.-%, vorzugsweise 0,03 bis 3 Gew.-%, insbesondere von 0,04 bis 1 Gew.-%, bezogen auf Gewicht an thermoplastischem Polycarbonat, zusammen mit Cyanursäure in Mengen von 0,0005 bis 2 Gew.-%, vorzugsweise von 0,0008 - 0,5 Gew.-%, bezogen wiederum auf Gewicht an thermoplastischem Polycarbonat, einsetzt.

Gemäß den US-Patenten 4 097 425 und 4 163 037 werden Dihydrooxadiazinone als Treibmittel für Polycarbonate eingesetzt, sowie gemäß US-Patent 4 288 560 Dioxazolone, wegen des im Vergleich zu den erfindungsgemäßen eingesetzten Treibmitteln auf Basis der Oxadiazolone der Formel I anderen Zersetzungsverhaltens der Dihydrooxadiazinone und Dioxazolone ist dort die vorliegende Problemstellung nicht angesprochen.

5-Phenyltetrazol (siehe beispielsweise US-Patent 4 263 409) und Benzazimide (US-Patent 3 779 954) sind ebenfalls als Treibmittel für Polycarbonate beschrieben, haben den Nachteil eines stärkeren negativen Einflusses auf den Molekulargewichtsabbau des Polycarbonats bei der Verschäumung.

Die erfindungsgemäß einzusetzenden Oxadiazolone der Formel I sind entweder bekannt oder nach bekannten Verfahren erhältlich.

Die Herstellung der bereits bekannten Verbindungen ist beschrieben in Chem. Ber. 82, S. 121-123 (1949) und in Tetrahedron Letters (44) S. 3875-3878 (1974). Die übrigen, noch nicht vorbeschriebenen, erfindungsgemäß einzusetzenden Oxadiazolone der Formel I lassen sich analog der in diesen Literaturstellen beschriebenen Verfahren herstellen.

Die Synthese erfolgt im einzelnen durch Umsetzung von Benzoesäurehydrazid mit Phosgen in einem geeigneten Lösungsmittel wie beispielsweise Methylenchlorid, Chlorbenzol, Toluol, Xylol, Wasser, Aceton, Chloroform, Tetrachlorethan oder deren Mischungen. Das draus resultierende 2-Phenyl-1-oxa-3,4-diazolon-(5) wird anschließend mit einem Äquivalent eines Monocarbonsäurechlorids oder Monochlorkohlensäureesters beziehungsweise mit einem halben Äquivalent eines Dicarbonsäurechlorids oder eines Dichlorkohlensäureesters in Gegenwart eines Säurefängers umsetzt.

Geeignete Monocarbonsäurechloride und Monochlorkohlensäureester sind solche der Formel II,

$$Cl-\underset{\underset{O}{\|}}{C}-R_1 \qquad (II)$$

worin $R_1$ die Bedeutung der einbindigen Reste R von Formel I hat;
geeignete Dicarbonsäurechloride und Dichlorkohlensäureester sind solche der Formel III,

$$Cl-\underset{\underset{O}{\|}}{C}-R_2-\underset{\underset{O}{\|}}{C}-Cl \qquad (III)$$

worin $R_2$ die Bedeutung der zweibindingen Reste R von Formel I hat oder eine Einfachbindung ist.

Besonders geeignete Carbonsäurechloride bzw. Chlorkohlensäureester der Formel II sind Benzoesäurechlorid, Naphthoesäurechloride, Chlorkohlensäure-phenylester, -ethylester und -propylester.

Besonders geeignete Dicarbonsäuredichloride bzw. Dichlorkohlensäureester der Formel III sind Isophthalsäuredichlorid, Terephthalsäuredichlorid, Bernsteinsäuredichlorid, Oxalsäuredichlorid, Malonsäuredichlorid und der Bis-chlorkohlensäureester des Bisphenol A.

Als Säurefänger bei der Reaktion des 2-Phenyloxadiazolons mit dem Säurechlorid können tertiäre Amine, wie beispielsweise Pyridin oder Triethylamin, oder Alkalimetallhydroxide dienen.

Erfindungsgemäß zu verschäumende, aromatische Polycarbonate sind Polykondensate die durch Umsetzung von Diphenolen mit Phosgen oder Diestern der Kohlensäure in bekannter Weise synthetisiert werden.

Geeignete Diphenole sind z. B. Hydrochinon, Resorcin, 4,4'-Dihydroxydiphenyl, Bis-(hydroxy-phenyl)-alkane wie beispielsweise Bis-(4-hydroxyphenyl)-propan-2,2 (Bisphenol A), Bis-(4-hydroxy-3,5-dimethylphenyl)-propan-2,2 (Tetramethylbisphenol A), halogenierte Bis-(hydroxyphenyl)alkane wie beispielsweise Bis-(4-hydroxy-3,5-dichlorphenyl)-propan-2,2 (Tetrachlorbisphenol A), Bis-(4-hydroxy-3,5-dibromphenyl)-propan-2,2 (Tetrabrombisphenol A), Dreikernbisphenole wie α,α'-Bis-(4-hydroxyphenyl)-p-diisopropylbenzol und Bis-(hydroxyphenyl)-sulfide, -sulfoxide, -sulfone oder -ether.

Weitere für die Herstellung von Polycarbonat geeignete Diphenole sind in den US-Patenten 3 028 365, 2 999 835, 3 148 172 und 2 999 846 beschrieben.

Die aromatischen Polycarbonate sollen in der Regel mittlere Gewichtsmittel-Molekulargewichte $\bar{M}_w$ von 10 000 bis über 200 000, vorzugsweise von 20 000 bis 80 000 haben, ermittelt durch Messungen der relativen Lösungsviskosität in $CH_2Cl_2$ bei 25° C und einer Konzentration von 0,5 g in 100 ml $CH_2Cl_2$.

Die erfindungsgemäß zu verschäumenden aromatischen, thermoplastischen Polycarbonate können linear oder verzweigt sein.

Die erfindungsgemäße Mitverwendung der Cyanursäure erlaubt nun, daß sich vorab lagerfähige Granulate von Treibmitteln der Formel I in thermoplastischen Polycarbonaten in Mengen von 1 bis 20 Gew.-%, vorzugsweise von 5 bis 10 Gew.-% bei Temperaturen von 200° C bis 270° C in üblichen Mischaggregaten wie Einwellenschneckenextrudern oder Zweiwellenschneckenextrudern oder Knetern in bekannter Weise herstellen lassen, welche als "Konzentrate" jeweils zusammen mit weiterem thermoplastischen Polycarbonat und der Cyanursäure gemäß dem erfindungsgemäßen Verfahren zu Polycarbonatschäumen bei Temperaturen von 270° C bis 320° C auf Spritzgußmaschinen in bekannter Weise verarbeiten lassen.

Die Zugabe der Cyanursäure kann auch in Form von Abmischungen im Polycarbonat erfolgen, welche bei üblichen Kompoundiertemperaturen für Polycarbonate vorab hergestellt werden können, wobei Gehalte an Cyanursäure im Polycarbonat von 0,0005 bis 5 Gew.-%, vorzugsweise von 0,0008 bis 2 Gew.-% üblich sind.

Derartige separate Abmischungen von Cyanursäure im Polycarbonat können nun entweder mit weiterem thermoplastischen Polycarbonat und dem Treibmittel-haltigen Polycarbonatgranulat oder gegebenenfalls nur mit dem Treibmittel-haltigen Polycarbonatgranulat oder gegebenenfalls nur mit Treibmittel für das erfindungsgemäße Verschäumungsverfahren eingesetzt werden.

Die Durchführung des erfindungsgemäßen Verschäumungsverfahrens kann natürlich auch dadurch erfolgen, daß man das Treibmittel und/oder die Cyanursäure in Substanz, also unverdünnt einsetzt.

Die zur Durchführung des erfindungsgemäßen Verfahrens einzusetzenden Komponenten, Treibmittel, Cyanursäure und thermoplastisches Polycarbonat, gegebenenfalls in Form der vollständigen oder partiellen separaten Abmischungen von Cyanursäure und/oder Treibmittel mit Polycarbonat, werden vor der

Verarbeitung zu Polycarbonatschaum beziehungsweise Polycarbonatschaumteilen in der üblichen Weise bei Raumtemperatur physikalisch innig vermischt.

Für die Herstellung eines besonders feinzelligen Schaums sind gewisse Mengen bis etwa 8 Gew.-%, bezogen auf Polycarbonatgewicht, beispielsweise an Glasfasern oder an mineralischen Füllstoffen als Nukleierungsmittel zweckmäßig.

Außer den erfindungsgemäß zu verwendenden Treibmitteln der Formel I, der Cyanursäure sowie den vorstehend genannten Glasfasern und Füllstoffen können den Polycarbonaten noch die üblichen Additive wie Stabilisatoren gegen Hitze und UV-Licht, Flammschutzmittel, Pigmente, Farbstoffe oder Gleitmittel sowie höhere Mengen an Glasfasern und Füllstoffen zugesetzt werden.

Die nach dem erfindungsgemäßen Verfahren verschäumten Polycarbonate können als Formteile bzw. Schaumteile überall dort eingesetzt werden, wo Polycarbonatschaum bislang verwendet wurde, so z. B. zur Herstellung großflächiger Abdeckungen von Lampen, Gehäusen, Bürogeräten und zur Herstellung großflächiger Schrankelemente.

**Beispiel 1**

Herstellung von Di-[2-phenyl-1,3,4-oxadiazolon-(5)]-4-terephthalamid

68 g (0,5 Mol) Benzhydrazid werden in 500 ml Wasser-Aceton suspendiert, bei 10° C - 20° C leitet man 55-60 g (ca. 10-20 Mol-% Überschuß) Phosgen ein. Durch gleichzeitiges Zutropfen von 45 %igem NaOH wird der pH-Wert auf etwa 2 bis 2,5 gehalten. Man rührt eine weitere halbe Stunde bei 20° C. Der Niederschlag wird abgesaugt, mit Wasser gewaschen und bei 100° C im Vakuum getrocknet. Das erhaltene 2-Phenyl-1,3,4-oxadiazolon-(5) wird in 500 ml Toluol vorgelegt, man gibt 50 g (0,6 Mol) Pyridin zu und tropft 51 g (0,25 Mol) Terephthalsäuredichlorid in 150 ml Toluol zu. Man rührt 45 Min. bei 25° C und erhitzt dann noch 1/2 h zum Rückfluß.

Nach dem Abkühlen gießt man in eine größere Menge Wasser, saugt den Niederschlag ab, wäscht mit Alkohol und Wasser chloridfrei. Trocknen bei 80-100° C im Vakuum.

Die Ausbeute beträgt 88 g = 78 % d. Th.

Fp: 282° C Zersetzungstemperatur: 285° C.

**Beispiel 2**

Die Gasabspaltung verschiedener Treibmittelgemische als Funktion der Temperatur wurde in eine Standard-Vorrichtung (Kolben, pneumatische Wanne) vorgenommen. Die Aufheizrate betrug 7,5° C/Min. Die Vorrichtung ist für vergleichende Untersuchungen geeignet, sie gibt nur relative, keine absoluten Werte an.

Untersucht wurden:

| | |
|---|---|
| Treibmittel 1: | 2 g Di-(2-phenyl-1,3,4-oxadiazolon-(5))-4-terephthalamid |
| Treibmittelgemisch 2: | 2 g Treibmittel 1<br>+ 0,02 g (= 1 %) Cyanursäure |
| Treibmittelgemisch 3: | 2 g Treibmittel 1<br>+ 0,1 (= 5 %) Cyanursäure |
| Treibmittelgemisch 4: | 2 g Treibmittel 1<br>+ 0,2 g (= 10 %) Cyanursäure. |

**Tabelle 1**

| °C | Treibmittel 1 ml | Treibmittelgemische 2 Gasmenge | 3 | 4 |
|---|---|---|---|---|
| 200 | 0 | 0 | 0 | 0 |
| 205 | 4 | 14 | 13 | 9 |
| 210 | 7 | 15 | 14 | 12 |
| 215 | 8 | 16 | 15 | 13 |
| 220 | 9 | 16 | 16 | 13 |
| 225 | 10 | 17 | 16 | 14 |
| 230 | 10 | 18 | 17 | 15 |
| 235 | 12 | 18 | 18 | 15 |
| 240 | 12 | 19 | 19 | 17 |
| 245 | 13 | 20 | 20 | 18 |
| 250 | 14 | 22 | 22 | 20 |
| 255 | 15 | 25 | 25 | 23 |
| 260 | 18 | 28 | 30 | 29 |
| 265 | 21 | 33 | 40 | 42 |
| 270 | 26 | 42 | 60 | 60 |
| 275 | 34 | 57 | 83 | 83 |
| 280 | 45 | 73 | 108 | 110 |
| 285 | 60 | 90 | 135 | 138 |
| 290 | 80 | 107 | 160 | 162 |
| 295 | 104 | 127 | 184 | 195 |
| 300 | 124 | 150 | 204 | 222 |
| 305 | 145 | 171 | 222 | 241 |
| 310 | 163 | 190 | 237 | 254 |
| 315 | 180 | 207 | 246 | 264 |
| 320 | 198 | 220 | 254 | 271 |

**Beispiel 3**

5 g Cyanursäure wurden auf 5000 g eines Bisphenol-A-Homopolycarbonats mit einer relativen Lösungsviskosität von 1,313 in einem Wirbelmischer aufgebracht und anschließend auf einem Entgasungsextruder einkompoundiert, wobei die einzelnen Heizzonen wie folgt eingestellt waren: 240°C (Einzugszone), 230°C, 240°C, 240°C, 230°C, 230°C, 220°C (Düse). Der austretende Schmelzstrang wurde zu Granulat geschnitten; das resultierende Granulat war fast völlig transparent und zeigt eine relative Lösungsviskosität von 1,307. Der Abfall der relativen Lösungsviskosität von 1,313 auf 1,307 bei einem einmaligen Kompoundierschnitt zeigt an, daß die Cyanursäure keinen nennenswerten Einfluß auf den Molekulargewichtsabbau des Polycarbonats hat, da eine derartige Abnahme der Lösungsviskosität bei einer Extrusion des Polycarbonats auch ohne Cyanursäureeinsatz auftreten kann.

**Beispiel 4**

Das in Beispiel 3 beschriebene Granulat aus Polycarbonat mit einem Gehalt von 0,1 Gew.-% Cyanursäure, bezogen auf Gewicht Polycarbonat, wurde nach Vortrocknung bei einer Massetemperatur von 290°C auf einer Anker Spritzgußmaschine zu Prüfkörpern der Dimension 60 mm x 6 mm x 4 mm verarbeitet.

Die am Prüfkörper gemessene relative Lösungsviskosität des Polycarbonats betrug 1,304. Es hat somit unter dieser thermo-mechanischen Belastung ein Abfall der relativen Lösungsviskosität von 1,307 auf 1,304 stattgefunden, was als typischer Abbau für Polycarbonat beim Spritzguß vorgang angesehen werden kann. Ein zusätzlicher Molekulargewichts-Abbau als Folge der Anwesenheit von 0,1 Gew.-% Cyanursäure tritt somit hierbei nicht auf.

**Beispiel 5**

100 g Bisphenol-A-Homopolycarbonat (relative Lösungsviskosität 1,310) mit einem Gehalt an 5 Gew.-% Glasfasern, bezogen auf Polycarbonat + Glasfasern, (Dichte des glasfaserhaltigen Polycarbonats = 1,24 g/cm³) wurden

a) mit 10 g eines Bisphenol-A-Homopolycarbonats (relative Lösungsviskosität 1,290), das 5 Gew.-% Treibmittel des Beispiels 1 eingearbeitet enthält, und

b) mit 10 g Cyanursäure-haltigem Polycarbonat des Beispiels 3 (relative Lösungsviskosität 1,307) versetzt und gut miteinander vermischt und nach Vortrocknung auf einer Anker Spritzgußmaschine bei einem Temperaturprofil von 250°C (Einzugszone), 300°C, 300°C, 300°C, 300°C (Düse) in einem wassergekühlten Werkzeug zu Rundplatten (200 mm Durchmesser, 8 mm dick, Dichte: 0,9 g/cm³) verarbeitet. Das Polycarbonat der Rundplatten zeigt eine relative Lösungsviskosität von 1,274.

Die Gesamtzykluszeit betrug 1,5 min. Es resultierten Formteile mit gleichmäßiger, feinzelliger Schaumstruktur und guter Oberfläche; das Formnest wurde vollständig gefüllt.

**Beispiel 6** (Vergleichsbeispiel)

Beispiel 5 wurde wiederholt, jedoch ohne Mitverwendung des Cyanursäurehaltigen Polycarbonats.

Das Polycarbonat der Rundplatten zeigt eine relative Lösungsviskotität von 1,275. Die Gesamtzykluszeit betrug 1,5 min. Die erhaltenen Formteile füllten das Formnest nicht vollständig aus, insbesondere am Fließwegende traten als Folge einer ungenügenden Treibwirkung deutliche Einfallstellen auf.

**Beispiel 7** (Vergleichsbeispiel)

Bisphenol-A-Homopolycarbonat (relative Lösungsviskosität 1,310) mit einem Gehalt an 5 Gew.-% Glasfasern, bezogen auf Polycarbonat + Glasfasern, wurde mit 0,4 Gew.-%, bezogen auf glasfaserhaltiges Polycarbonat, an 5-Phenyltetrazol in einem Taumelmischer vermischt und anschließend bei einem Temperaturprofil des Plastifizieraggregats von 200°C (Einzugszone), 250°C, 280°C, 300°C, 300°C (Düse) zu Rundplatten der Dimension 200 mm x 8 mm und einer Dichte von 0,9 g/cm³ verspritzt. Das Polycarbonat der Rundplatten zeigt eine relative Lösungsviskosität von 1,251.

**Beispiel 8** (Vergleichsbeispiel)

Beispiel 7 wird wiederholt, wobei anstelle von 0,4 Gew.-% 5-Phenyltetrazol 0,5 Gew.-%, bezogen auf glasfaserhaltiges Polycarbonat, Treibmittel des Beispiels 1 zugemischt wird. Anschließend muß bei einem Temperaturprofil des Plastifizieraggregats von 200°C (Einzugszone), 250°C, 300°C, 320°C, 320°C (Düse) zu Rundplatten (200 mm x 8 mm) der Dichte 0,9 g/cm³ verspritzt werden, um eine ausreichende Formnestfüllung und Formteile ohne Einfallstellen zu erreichen. Das Polycarbonat der Rundplatten zeigte eine relative Lösungsviskosität von 1,264. Trotz der erforderlichen höheren Verarbeitungstemperatur ist der Abbau des Polycarbonats geringer als im Beispiel 7.

**Patentansprüche**

1. Verfahren zum Verschäumen von aromatischen, thermoplastischen Polycarbonaten bei Temperaturen von 270°C bis 320°C, welche gegebenenfalls noch Nukleierungsmittel enthalten, dadurch gekennzeichnet, daß man Verbindungen der Formel I

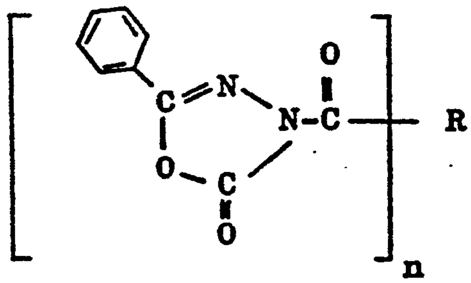

(I)

worin n = 1 und R $C_1$-$C_{18}$-Alkoxy, $C_6$-$C_{12}$-Cyclo-alkoxy, $C_6$-$C_{18}$-Aryl, $C_6$-$C_{12}$-Aryloxy, $C_7$-$C_{18}$-Aralkyl, $C_7$-$C_{18}$-Aralkoxy, $C_7$-$C_{18}$-Alkaryl und $C_7$-$C_{18}$-Alkaryloxy bedeuten, oder worin n = 2 und R $C_6$-$C_{18}$-Arylen, $C_6$-$C_{18}$-Arylen-dioxy, Isopropyliden-bis-(phenylenoxy) und $C_1$-$C_5$-Alkylen bedeuten oder worin n = 2 und R eine Einfachbindung ist, und die außerdem eine Zersetzungstemperatur in Substanz von mindestens 265° C haben müssen, in Mengen von 0,02 bis 5 Gew.-%, bezogen auf Gewicht an thermoplastischem Polycarbonat, zusammen mit Cyanursäure in Mengen von 0,0005 bis 2 Gew.-%, bezogen wiederum auf Gewicht an thermoplastischem Polycarbonat einsetzt.

2. Verfahren nach Anspruch 1, dadurch gekennzeichnet, daß man die Verbindungen der Formel I in Mengen von 0,03 bis 3 Gew.-% einsetzt.

3. Verfahren nach Ansprüchen 1 und 2, dadurch gekennzeichnet, daß man die Cyanursäure in Mengen von 0,0008 bis 0,5 Gew.-% einsetzt.

**Claims**

1. Process for foaming aromatic thermoplastic polycarbonates at temperatures of 270° C to 320° C, which possibly still contain nucleating agents, characterised in that compounds of the formula I

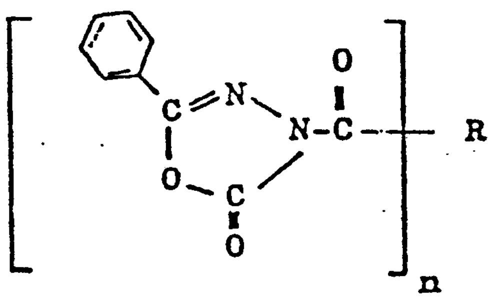

(I)

wherein
n denotes 1 and
R denotes $C_1$-$C_{18}$-alkoxy, $C_6$-$C_{12}$-cycloalkoxy, $C_6$-$C_{18}$-aryl, $C_6$-$C_{12}$-aryloxy, $C_7$-$C_{18}$-aralkyl, $C_7$-$C_{18}$-aralkoxy, $C_7$-$C_{18}$-alkaryl and $C_7$-$C_{18}$-alkaryloxy,
or wherein
n denotes 2 and
R denotes $C_6$-$C_{18}$-arylene, $C_6$-$C_{18}$-arylene-dioxy, isopropylidene-bis-(phenyleneoxy) and $C_1$-$C_5$-alkylene
or wherein
n = 2 and R is a single bond,
and which must also have a decomposition temperature in bulk of at least 265° C, are used, in quantities of 0.02 to 5 % by weight, based on the weight of thermoplastic polycarbonate, together with cyanuric acid in quantities of 0.0005 to 2 % by weight, again based on the weight of thermoplastic polycarbonate.

2. Process according to Claim 1, characterised in that the compounds of the formula I are used in quantities of 0.03 to 3 % by weight.

3. Process according to Claims 1 and 2, characterised in that cyanuric acid is used in quantities of 0.0008 to

0.5 % by weight.

## Revendications

1. Procédé pour transformer en mousse à des températures de 270 à 320° C, des polycarbonates aromatiques thermoplastiques qui contiennent encore, le cas échéant, des agents de nucléation, caractérisé en ce qu'on utilise des composés de formule I

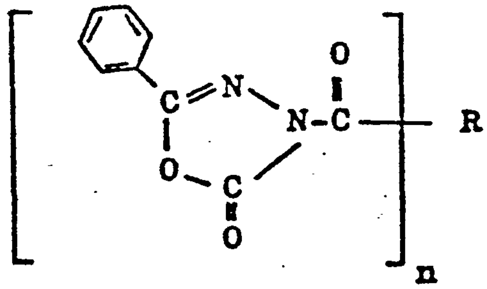

(I)

dans laquelle n est égal à 1 et R est un groupe alkoxy en $C_1$ à $C_{18}$, cycloalkoxy en $C_6$ à $C_{12}$, aryle en $C_6$ à $C_{18}$, aryloxy en $C_6$ à $C_{12}$, aralkyle en $C_7$ à $C_{18}$, aralkoxy en $C_7$ à $C_{18}$, alkaryle en $C_7$ à $C_{18}$ et alkaryloxy en $C_7$ à $C_{18}$, ou bien n est égal à 2 et R est un groupe arylène en $C_6$ à $C_{18}$, arylènedioxy en $C_6$ à $C_{18}$, isopropylidène-bis-(phénylèneoxy) et alkylène en $C_1$ à $C_5$, ou encore n est égal à 2 et R est une liaison simple, et qui doivent en outre avoir, en substance, une température de décomposition d'au moins 265° C, en quantités de 0,02 à 5 % en poids sur la base du poids de polycarbonate thermoplastique, conjointement avec de l'acide cyanurique en quantités de 0,0005 à 2 % en poids, là encore par rapport au poids de polycarbonate thermoplastique.

2. Procédé selon la revendication 1, caractérisé en ce qu'on utilise les composés de formule I en quantités de 0,03 à 3 % en poids.

3. Procédé suivant les revendications 1 et 2, caractérisé en ce qu'on utilise l'acide cyanurique en quantités de 0,0008 à 0,5 % en poids.